# EUROPEAN PATENT APPLICATION

(11) **EP 2 053 046 A1**
(43) Date of publication of application: **29.04.2009**
(21) Application number: 08007093.1
(22) Date of filing: 10.04.2008
(51) Int. Cl.: C07D 233/54, C07D 233/61, C07D 233/68, C07D 409/04, C07D 409/14, A01N 43/50

(54) **Novel imidazole derivatives**

(30) Priority: 26.10.2007 EP 07020978; 17.12.2007 EP 07024448
(71) Applicant: Syngenta Participations AG, 4058 Basel (CH)
(72) Inventor: The designation of the inventor has not yet been filed
(74) Representative: Hölscher, Ingo

(57) **Abstract**

The present invention relates to novel imidazole derivatives of formula I as active ingredients which have microbiocidal activity, in particular fungicidal activity: 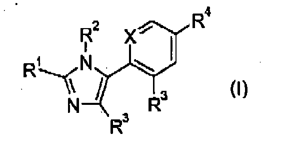 wherein
R¹ is halogen, C₁-C₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, hydroxyl. C₁-C₄alkoxy, OR⁶, C₁-C₄haloalkoxy or cyano;
R⁵ is halogen;
R⁶ is hydrogen, C₃-C₇ cycloalkyl, C₃-C₁₀ alkylcycloalkyl, C₁-C₆ haloalkyl, C₂-C₆ alkenyl, C₂-C₆ haloalkenyl, C₃-C₇ cycloalkenyl, C₂-C₆ alkynyl, C₂-C₆ haloalkynyl or C₂-C₆ alkyloxyalkyl;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;
provided that
when X is C(R), R² cannot be an optionally substituted aryl.

## Description

The present invention relates to novel imidazole derivatives as active ingredients which have microblocidal activity, in particular fungicidal activity. The invention also relates to preparation of these active ingredients, to novel heterocyclic derivatives used as intermediates in the preparation of these active ingredients, to preparation of these novel intermediates, to agrochemical compositions which comprise at least one of the novel active ingredients, to preparation of these compositions and to use of the active ingredients or compositions in agriculture or horticulture for controlling or preventing infestation of plants, harvested food crops, seeds or non-living materials by phytopathogenic microorganisms, preferably fungi.

The present invention provides a compound of formula **I**: 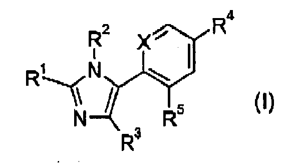 wherein
R¹ is halogen, C₁-C₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, hydroxyl, C₁-C₄alkoxy, OR⁶, C₁-C₄haloalkoxy or cyano;
R⁵ is halogen;
R⁶ is hydrogen, C₃-C₇ cycloalkyl, C₃-C₁₀ alkylcycloalkyl, C₁-C₆ haloalkyl, C₂-C₆ alkenyl, C₂-C₆ haloalkenyl, C₃-C₇ cycloalkenyl, C₂-C₆ alkynyl, C₂-C₆ haloalkynyl or C₂-C₆ alkyloxyalkyl;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;
provided that
when X is C(R), R² cannot be an optionally substituted aryl.

In the above definition aryl includes aromatic hydrocarbon rings like phenyl, naphthyl, anthracenyl, phenanthrenyl and biphenyl, with phenyl being preferred.

Heteroaryl stands for aromatic ring systems comprising mono-, bi- or tricyclic systems wherein at least one oxygen, nitrogen or sulfur atom is present as a ring member. Examples are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolyl, isoquinolyl, phthalazinyl, quinoxalinyl, quinazolinyl, cinnolinyl and naphthyridinyl.

The above or below mentioned fused ring, carbocyclic ring, heterocyclic ring, aryl group and heteroaryl group may be optionally substituted. This means that they may carry one or more identical or different substituents. Normally not more than three substituents are present at the same time. Examples of substituents are: halogen, alkyl, haloalkyl, cycloalkyl, cycloalkylalkyl, alkenyl, haloalkenyl, cycloalkenyl, alkynyl, haloalkynyl, alkyloxy, haloalkyloxy, cycloalkoxy, alkenyloxy, haloalkenyloxy, alkynyloxy, haloalkenyloxy, alkylthio, haloalkylthio, cycloalkylthio, alkenylthio, alkynylthio, alkylcarbonyl, haloalkylcarbonyl, cycloalkylcarbonyl, alkenylcarbonyl, alkynylcarbonyl, alkoxyalkyl, cyano, nitro, hydroxy, mercapto, amino, alkylamino, dialkylamino. Typical examples for optionally substituted aryl include 2-fluorophenyl, 3-fluorophenyl, 4-fluorophenyl, 2-chlorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl. 3-methoxyphenyl, 4-methoxyphenyl, 3-trifluoromethoxyphenyl, 4-trifluoromethoxyphenyl, 3-cyanophenyl, 4-cyanophenyl, 2,4-difluorophenyl, 2,5-difluorophenyl, 2,6-difluorophenyl, 3,4-difluorophenyl, 2,4-dichlorophenyl, 2,5-dichlorophenyl, 2,6-dichlorophenyl, 3,4-dichlorophenyl, 3,4-dimethylphenyl, 3,4-dimethoxyphenyl, 2-chloro-4-fluorophenyl, 2-chloro-5-fluorophenyl, 2-chloro-6-fluorophenyl, 3-chloro-4-fluorophenyl, 3-chloro-6-fluorophenyl, 3-chloro-4-methylphenyl, 3-chloro-4-methoxyphenyl, 4-chloro-2-fluorophenyl, 4-chloro-3-fluorophenyl, 4-chloro-3-methylphenyl, 4-chloro-3-methoxyphenyl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, 2,6-difluoro-4-methylphenyl, 2,6-difluoro-4-trifluoromethylphenyl, 2,6-difluoro-4-methoxyphenyl, 2,6-difluoro-4-trifluoromethoxyphenyl, 2,6-difluoro-4-cyanophenyl, 2,4,6-trifluorophenyl, 2,5,6-trifluorophenyl. Typical examples for optionally substituted heteroaryl include 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 3,5-dichloropyridin-2-yl, 3,5-difluoropyridin-2-yl, 3-chloro-5-fluoropyridin-2-yl, 3-chloro-5-methylpyridin-2-yl, 3-chloro-5-trfluoromethylpyridin-2-yl, 3-chloro-5-methoxypyridin-2-yl, 3-chloro-5-trifluoromethoxypyridin-2-yl, 3-chloro-5-cyanopyridin-2-yl, 5-chloro-3-fluoropyridin-2-yl, 3-fluoro-5-methylpyridin-2-yl, 3-fluoro-5-trifluoromethylpyridin-2-yl, 3-fluoro-5-methoxypyridin-2-yl, 3-fluoro-5-trifluoromethoxypyridin-2-yl, 3-fluoro-5-cyanopyridin-2-yl, 5-chlorothiophen-2-yl, 5-bromothiophen-2-yl, 5-methoxythiophen-2-yl, 4-methoxyquinolin-2-yl, 4-methylquinolin-2-yl.

In the above definition halogen is fluorine, chlorine, bromine or iodine.

The alkyl, alkenyl or alkynyl radicals may be straight-chained or branched.

Alkyl on its own or as part of another substituent is, depending upon the number of carbon atoms mentioned, for example, methyl, ethyl, propyl, butyl, pentyl, hexyl and the isomers thereof, for example, isopropyl, isobutyl, sec-butyl, tert-butyl, isopentyl or tert-pentyl.

A haloalkyl group may contain one or more identical or different halogen atoms and, for example, may stand for CH₂Cl, CHCl₂, CCl₃, CH₂F, CHF₂, CF₃, CF₃CH₂, CH₃CF₂, CF₃CF₂ or CCl₃CCl₂.

Cycloalkyl on its own or as part of another substituent is, depending upon the number of carbon atoms mentioned, for example, cyclopropyl, cyclobutyl, cyclopentyl or cyclohexyl.

Alkenyl on its own or as part of another substituent is, depending upon the number of carbon atoms mentioned, for example, ethenyl, allyl, 1-propenyl, buten-2-yl, buten-3-yl, penten-1-yl, penten-3-yl, hexen-1-yl or 4-mothyl-3-pentenyl,

Alkynyl on its own or as part of another substituent is, depending upon the number of carbon atoms mentioned, for example, ethynyl, propyn-1-yl, propyn-2-yl, butyn-1-yl, butyn-2-yl, 1-methyl-2-butynyl, hexyn-1-yl or 1-ethyl-2-butynyl.

The presence of one or more possible asymmetric carbon atoms in a compound of formula I means that the compounds may occur in optically isomeric, that means enantiomeric or diastereomeric forms. As a result of the presence of a possible aliphatic C=C double bond, geometric isomerism, that means cis-trans or (*E*)-(*Z*) isomerism may also occur. Also atropisomers may occur as a result of restricted rotation about a single bond. Formula I is intended to include all those possible isomeric forms and mixtures thereof. The present invention intends to include all those possible isomeric forms and mixtures thereof for a compound of formula I.

in each case, the compounds of formula I according to the invention are in free form or in an agronomically usable salt form.

In a first embodiment, compounds of formula I according to the invention have R¹ which is halogen, C₁-C₃alkyl or C₁-C₃haloalkyl.

In a second embodiment, compounds of formula I according to the invention have R² which is an optionally substituted phenyl, naphthyl, thienyl, pyridyl or quinolyl.

In a third embodiment, compounds of formula I according to the invention have R³ which is fluoro, chlbro or bromo.

In a fourth embodiment, compounds of formula I according to the invention have R⁴ which is hydrogen, halogen, C₁-C₄alkyl, C₁-C₄holoalkyl, C₁-C₄alkoxy, OR⁶, C₁-C₄haloalkoxy or cyano.

In a fifth embodiment, compounds of formula I according to the invention have R⁵ which is fluoro, chloro, bromo or iodo.

In a sixth embodiment, compounds of formula I according to the invention have R⁶ which is hydrogen, C₃-C₇ cycloalkyl, C₃-C₁₀ alkylcycloalkyl, C₁-C₆ haloalkyl, C₂-C₆ alkenyl, C₃-C₇ cycloalkenyl, C₂-C₆ alkynyl, or C₂-C₆ alkyloxyalkyl.

In a seventh embodiment, compounds of formula I according to the invention have X which is N, C(H), C(halogen), C(C₁-C₄alkyl), C(C₁-C₄haloalkyl), C(C₁-C₄alkoxy) or C(C₁-C₄haloalkoxy).

Preferred subgroups of compounds of formula I according to the invention are those wherein
R¹ is fluoro, chloro, bromo, iodo, C₁-C₂alkyl or C₁-C₂haloalkyl;
R² is an optionally substituted phenyl, naphtyl, thienyl, pyridyl or quinolyl;R³ is fluoro, chloro or bromo;
R⁴ is hydrogen, fluoro, chloro, bromo, C₁-C₃alkyl, C₁-C₃haloalkyl, C₁-C₃alkoxy, C₁-C₃haloalkoxy or cyano;
R⁵ is fluoro, chloro or bromo; and
X is N, C(H), C(Cl), C(F), C(Br), C(I), C(C₁-C₃alkyl), C(C₁-C₃haloalkyl), C(C₁-C₃alkoxy) or C(C₁-C₃haloalkoxy).

More preferred subgroups of compounds of formula I according to the invention are those wherein
R¹ is fluoro, chloro, bromo, methyl or ethyl;
R² is phenyl, 3-fluorophenyl, 4-fluorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl, 3-methoxyphenyl, 4-methoxyphenyl, 3-trifluoromethoxyphenyl, 4-trifluoromethoxyphenyl, 3-cyanophenyl, 4-cyanophenyl, 3,4-difluorophenyl, 3,4-dichlorophenyl, 3,4-dimethylphenyl, 3,4-dimethoxyphenyl, 3-chloro-4-fluorophenyl, 3-chloro-4-methylphenyl, 3-chloro-4-methoxyphenyl, 4-chloro-3-fluorophenyl, 4-chloro-3-methylphenyl, 4-chloro-3-methoxyphenyl, naphth-2-yl, 3-fluoro-4-methoxyphenyl, 3-fluoro-4-methylphenyl, 4-fluoro-3-mothoxyphenyl, 4-fluoro-3-methylphenyl, 3-methoxy-4-methylphenyl, 4-methoxy-3-methylphenyl, pyridin-2-yl, pyridin-3-yl, pyridin-4-yl, 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 5-chlorothiophen-2-yl, 5-bromothiophen-2-yl, 5-methoxythiophen-2-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl, C₁-C₂alkoxy, C₁-C₂haloalkoxy or cyano;
R⁵ is fluoro or chloro; and
X is N, C(H), C(Cl), C(F), C(Br), C(C₁-C₂alkyl), C(C₁-C₂haloalkyl), C(C₁-C₂alkoxy) or C(C₁-C₂haloalkoxy).

Most preferred subgroups of compounds of formula I according to the invention are those wherein
R¹ is fluoro, chloro, methyl or ethyl;
R² is 4-fluorophenyl, 4-chlorophenyl, 4-bromophenyl, p-tolyl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-mothoxypyridin-4-yl, 2-methylpyridin-4-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl or C₁-C₂alkoxy;
R⁵ is fluoro or chloro; and
X is N, C(H), C(Cl) or C(F).

Especially preferred subgroups of compounds of formula I according to the invention are those wherein
R¹ is chloro or methyl;
R² is 6-chloropyridin-3-yl, 6-methylpyridin-3-yl, 6-methoxypyridin-3-yl or quinolin-3-yl;
R³ is chloro;
R⁴ is fluoro or methoxy; and
R⁵ is fluoro; and
X is C(F).

Preferred individual compounds are:
2-chloro-5-[2,4-dichloro-5-(2,4,6-trifluoro-phonyl)-imidazol-1-yl]-pyridine;
2-chloro-5-[4-chloro-2-methyl-5-(2,4,6-trifluoro-phenyl)-imidazol-1-yl]-pyridine;
5-[4-chloro-2-methyl-5-(2,4,6-trifluoro-phenyl)-imidazol-1-yl]-2-methoxy-pyridine;
2-[4-chloro-2-methyl-5-(2,4,6-trifluoro-phenyl)-imidazol-1-yl]-quinoline;
2-[4-chloro-2-methyl-5-(2,4,5-trifluoro-phenyl)-imidazol-1-yl]-4-methoxy-quinoline;
3-[4-chloro-2-methyl-5-(2,4,6-trifluoro-phenyl)-imidazol-1-yl]-quinoline;
3-[2,4-dichloro-5-(2,4,6-trifluoro-phenyl)-imidazol-1-yl]-quinoline;
3-[2,4-dichloro-5-(2,6-difluoro-4-methoxy-phenyl)-imidazol-1-yl]-quinoline;
2-[4-chloro-5-(2,6-difluoro-4-methoxy-phenyl)-2-methyl-imidazol-1-yl]-4-methoxy-quinoline;
3-[4-chloro-5-(2,6-difluoro-4-methoxy-phenyl)-2-methyl-imidazol-1-yl]-quinoline;
2-chloro-5-[4-chloro-5-(2,6-difluoro-4-methoxy-phenyl)-2-methyl-imidazol-1-yl]-pyridine;
2-chloro-5-[2,4-dichloro-5-(2,6-difluoro-4-methoxy-phenyl)-imidazol-1-yl]-pyridine;
5-[4-chloro-5-(2,6-difluoro-4-methoxy-phenyl)-2-methyl-imidazol-1-yl]-2-methoxy-pyridine;
5-[4-chloro-5-(2,6-difluoro-4-methoxy-phenyl)-2-methyl-imidazol-1-yl]-2-methyl-pyridine;
3,5-dichloro-2-[5-chloro-3-(6-chloro-pyrldin-3-yl)-2-methyl-3H-imidazol-4-yl]-pyridine; and
2-[4-chloro-5-(3,5-dichloro-pyridin-2-yl)-2-methyl-imidazol-1-yl]-quinoline.

The compounds of formula I.1, wherein R², R³, R⁴ and X are as defined for compound of formula I, and R¹ is C₁-C₄alkyl or C₁-C₄haloalkyl, can be obtained by reaction of a compound of formula II, wherein R², R⁴, R⁵ and X are as defined for compound of formula I, and R¹ is C₁-C₄alkyl or C₁-C₄haloalkyl, with N-chlorosuccinimide or molecular chlorine.

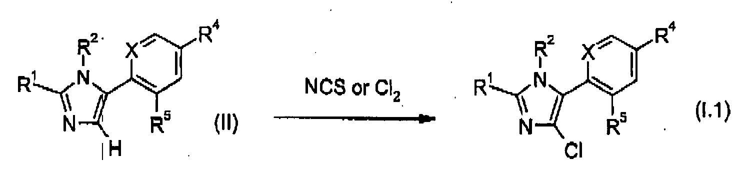

The compounds of formula I.2, wherein R² and R⁶ are as defined for compound of formula I, and R¹ C₁-C₄alkyl, can be obtained by reaction of a compound of formula 1.3, wherein R² is as defined for compound of formula I, and R¹ is C₁-C₄alkyl, with a reagent of formula NaOR⁶, wherein R⁶ is as defined for compound of formula I.

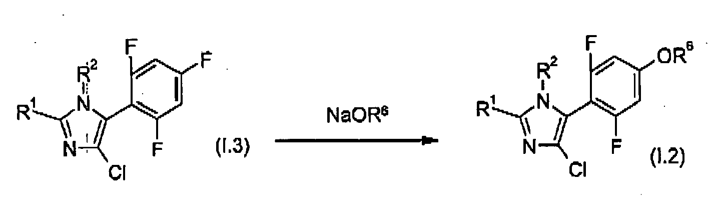

The compounds of formula II, wherein R², R⁴, R⁵ and X are as defined for compound of formula I, and R¹ is C₁-C₄alkyl, can be obtained by transformation of a compound of formula III, wherein R², R⁴, R⁵ and X are as defined for compound of formula I, with a reagent of formula (R¹)₃Al, wherein R¹ is C₁-C₄alkyl, preferably methyl, in the presence of a transition metal catalyst.

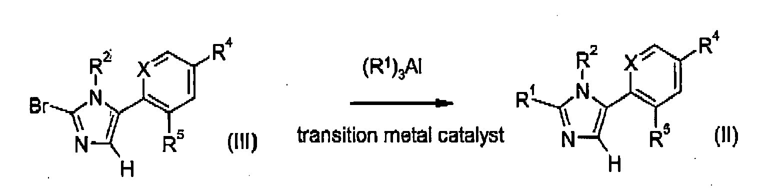

The compounds of formula II, wherein R², R⁴, R⁵ and X are as defined for compound of formula I, and R¹ is C₁-C₄alkyl or C₁-C₄haloalkyl, can alternatively be obtained by transformation of a compound of formula IV, wherein R², R⁴, R⁵ and X are as defined for compound of formula I, with a strong base, e.g. lithium di-isopropylamide, followed by a reagent of formula R¹Hal, wherein R¹ is C₁-C₄alkyl or C₁-C₄haloalkyl and Hal is halogen, preferably bromine or iodine.

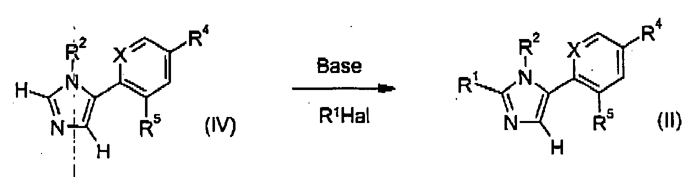

The compounds of formula I, wherein R², R⁴, R⁵ and X are as defined for compound of formula I, and R¹ is Halogen, preferably chlorine or bromine, can be obtained by reaction of a compound of formula IV, wherein R², R⁴, R⁵ and X are as defined for compound of formula I, with at least 2 equivalents of N-chlorosuccinimide, N-bromosuccinimide, molecular chlorine or bromine.

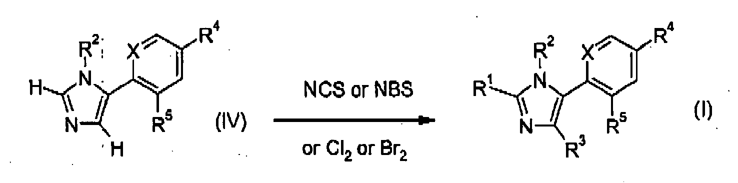

The compounds of formula III, wherein R², R⁴, R⁵ and X are as defined for compound of formula I, can be obtained by transformation of a compound of formula IV, wherein R², R⁴, R⁵ and X are as defined for compound of formula I, with N-bromosuccinimide.

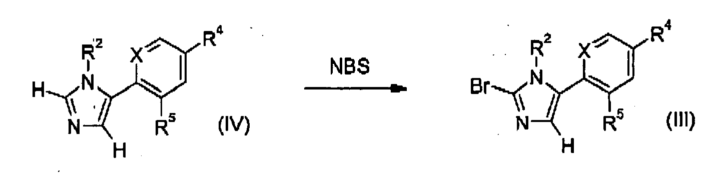

The compounds of formula IV, wherein R², R⁴, R⁵ and X are as defined for compound of formula I, can be obtained by reaction of a compound of formula V, wherein R², R⁴, R⁵ and X are as defined for compound of formula I, with toluenesulfonylmethyl isocyanide in the presence of a base, e.g. anhydrous potassium carbonate, as already described in Journal of Medicinal Chemistry 2003, 46, 3463.

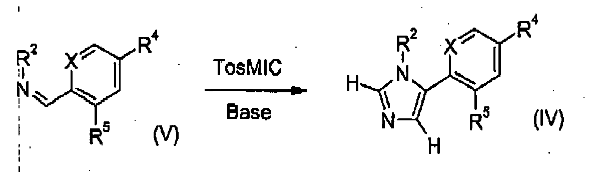

The compounds of formula V, wherein R², R⁴, R⁵ and X are as defined for compound of formula I, can be obtained by reaction of an aldehyde of formula VI, wherein R⁴, R⁵ and X are as defined for compound of formula I, with an amine of formula VII, wherein R² is as defined for compound of formula I, as already described in Journal of Medicinal Chemistry 2003, 46, 3463.

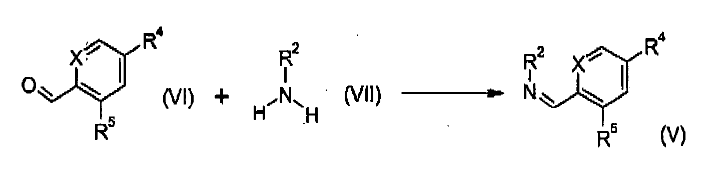

Surprisingly, it has now been found that the novel compounds of formula I have, for practical purposes, a very advantageous level of biological activity for protecting plants against diseases that are caused by fungi as well as by bacteria and viruses.

The compounds of formula I can be used in the agricultural sector and related fields of use as active ingredients for controlling plant pests or on non-living materials for control of spoilage microorganisms or organisms potentially harmfull to man. The novel compounds are distinguished by excellent activity at low rates of application, by being well tolerated by plants and by being environmentally safe. They have very useful curative, preventive and systemic properties and are used for protecting numerous cultivated plants. The compounds of formula I can be used to inhibit or destroy the pests that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula I as dressing agents for the treatment of plant propagation material, e.g., seed, such as fruits, tubers or grains, or plant cuttings (for example rice), for the protection against fungal infections as well as against phytopathogenic fungi occurring in the soil. The propagation material can be treated with a composition comprising a compound of formula I before planting: seed, for example, can be dressed before being sown. The active ingredients according to the invention can also be applied to grains (coating), either by impregnating the seeds in a liquid formulation or by coating them with a solid formulation. The composition can also be applied to the planting site when the propagation material is being planted, for example, to the seed furrow during sowing The invention relates also to such methods of treating plant propagation material and to the plant propagation material so treated.

Furthermore the compounds according to present invention can be used for controlling fungi in related areas, for example in the protection of technical materials including wood and wood related technical products, in food storage, in hygiene management.

In addition, the invention could be used to protect non-living materials from fungal attack, e.g. lumber wall boards and paint.

The compounds of formula I are, for example, effective against the phytopathogenic fungi of the following classes: Fungi imperfecti (e.g. *Alternaria* spp.), Basidiomycetes (e.g. *Corticium* spp., *Ceratobasidium* spp., *Waitea* spp., *Thanatephorus* spp., Rhizoctonia spp., *Hemileia* spp., *Puccinia* spp., *Phakopsora* spp., *Ustilago* spp., *Tilletia* spp.), Ascomycetes (e.g. *Venturia* spp., Blumeria spp., *Erysiphe* spp., *Podosphaera* spp., *Uncinula* spp., *Monilinia* spp., *Sclerotinia* spp., *Colletotrichum* spp., *Glomerella* spp., *Fusarium* spp., *Gibberella* spp., *Monographella* spp., *Phaeosphaeria* spp., *Mycosphaerella* spp., Cercospora spp., *Pyrenophora* spp., *Rhynchosporium* spp., *Magnaporthe* spp., *Gaeumannomyces* spp., *Oculimacula* spp., *Ramularia* spp., *Botryotinia* spp.) and Oomycetes (e.g. *Phytophthora* spp., *Pythium* spp., *Plasmopara* spp., *Peronospora* spp., *Pseudoperonospora* spp. *Bremia* spp). Outstanding activity has been observed against powdery mildews (e.g. *Uncinula necator),* rusts (e.g. *Puccinia* spp.) and leaf spots (e.g. *Mycosphaerella* spp.). Furthermore, the novel compounds of formula I are effective against phytopathogenic gram negative and gram positive bacteria (e.g. *Xanthomonas* spp, *Psoudomonas* spp, *Erwinia amylovora, Ralstonia* spp.) and viruses (e.g. tobacco mosaic virus).

Within the scope of present invention, target crops to be protected typically comprise the following species of plants: cereal (wheat, barley, rye, oat, rice, maize, sorghum and related species); beet (sugar beet and fodder beet); pomes, drupes and soft fruit (apples, pears, plums, peaces, almonds, cherries, strawberries, raspberries and blackbenies); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as turf and ornamentals.

The target crops in accordance with the invention include conventional as well as genetically enhanced or engineered varieties such as, for example, insect resistant (e.g. Bt. and VIP varieties) as well as disease resistant, herbicide tolerant (e.g. glyphosate- and glufosinate-resistant maize varieties commercially available under the trade names RoundupReady® and LibertyLink®) and nematode tolerant varieties. By way of example, suitable genetically enhanced or engineered crop varieties include the Stoneville 5599BR cotton and Stoneville 4892BR cotton varieties.

The compounds of formula I are used in unmodified form or, preferably, together with the adjuvants conventionally employed in the art of formulation. To this end they are conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula I are normally used in the form of compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula I are normally used in the form of fungicidal compositions for controlling or protecting against phytopathogenic microorganisms, comprising as active ingredient at least one compound of formula I, in free form or in agrochemically usable salt form, and at least one of the above-mentioned adjuvants.

The compounds of formula I can be mixed with other fungicides, resulting in some cases in unexpected synergistic activities. Mixing components which are particularly preferred are:
Azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, prothioconazole, pyrifenox, prochloraz, propiconazole, simeconazole, tebuconazole, tetraconazole, triadimefon, triadimenol, triflumizole, triticonazole;
Pyrimidinyl carbinoles, such as ancymidol, fenarimol, nuarimol;
2-amino-pyrimidines, such as bupirimate, dimethirimol, ethirimol;
Morpholines, such as dodemorph, fenpropidine, fenpropimorph, spiroxamine, tridemorph;
Anilinopyrimidines, such as cyprodinil, mepanipyrim, pyrimethanil;
Pyrroles, such as fenpiclonil, fludioxonil;
Phenylamides, such as benalaxyl, furalaxyl, metalaxyl, R-metalaxyl, ofurace, oxadixyl;
Benzimidazoles, such as benomyl, carbendazim, debacarb, fuberidazole, thiabendazole;
Dicarboximides, such as chlozolinate, dichlozoline, iprodione, myclozoline, procymidone, vinclozoline;
Carboxamides, such as boscalid, carboxin, fenfuram, flutolanil, mepronil, oxycarboxin, penthiopyrad, thifluzamide; guanidines, such as guazatine, dodine, iminoctadine;
Strobilurines, such as azoxystrobin, dimoxystrobin, enestroburin, fluoxastrobin, kresoxim-methyl, metominostrobin, trifloxystrobin, orysastrobin, picoxystrobin, pyraclostrobin;
Dithiocarbamates, such as ferbam, mancozeb, maneb, metiram, propineb, thiram, zineb, ziram;
N-halomethylthiotetrahydrophthalimides, such as captafol, captan, dichlofluanid, fluoromides, folpet, tolyfluanid;
Cu-compounds, such as Bordeaux mixture, copper hydroxide, copper oxychloride, copper sulfate, cuprous oxide, mancopper, oxine-copper;
Nitrophenol-derivatives, such as dinocap, nitrothal-isopropyl;
Organo-phosphorus-derivatives, such as edifenphos, iprobenphos, isoprothiolane. phosdiphen, pyrazophos, tolclofos-methyl;
Pyndazine-derivatives which are known and may be prepared by methods as described in WO 05/121104, WO 06/001175 and WO 07/066601, such as 3-chloro-5-(4-chloro-phenyl)-6-methyl-4-(2,4,6-trifluoro-phenyl)-pyridazine (formula P.1), 3-chloro-6-methyl-5-p-tolyl-4-(2,4,6-trifluoro-phenyl)-pyridazine (formula P.2) and 3-chloro-4-(3-chloo-5-methoxy-pyridin-2-yl)-5-(4-chloro-phenyl)-6-methyl-pyridazine (formula P.3); 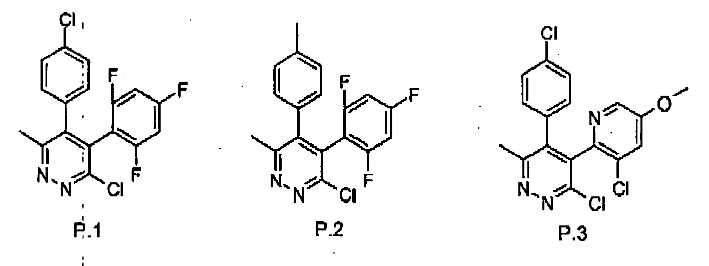
Triazolopyrimidine derivatives which are known and may be prepared by methods as described in WO98/46607. such as 5-chloro-7-(4-methyl-piperidin-1-yl)-6-(2,4,6-trifluoro-phenyl)- [1,2,4]triazolo[1,5-a]pyrimidine (formula T.1); 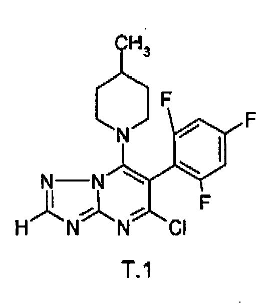
Carboxamide derivatives which are known and may be prepared by methods as described in WO04/035589 and in WO06/37632, such as 3-difluoromethyl-1-methyl-1H-pyrazole-4-carboxylic acid (9-isopropyp-1,2,3,4-tetrahaydro-1,4-methano-naphthalen-6-yl)-amide (formula U.1); or 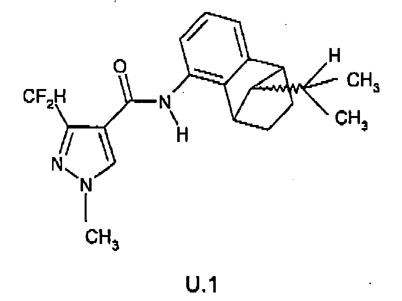 N-(3',4'-dichloro-5-fluoro-1,1'-biphenyl-2-yl)-3-(difluoromethyl)-1-methyl-1H-pyrazole-4-carboxamide (compound F-13)
Benzamide derivatives which are known and may be prepared by methods as described in WO 2004/016088, such as N-{-2-[3-chloro-5-(bifluoromethyl)-2-pyridinyl]ethyl}-2-trifluoromethylbenzamide, which is also known under the name fluopyram (formula V.1); and 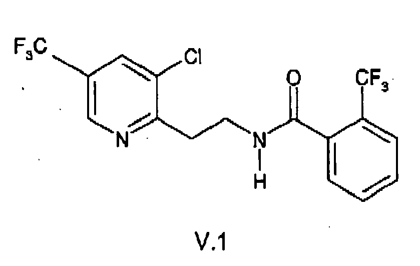
Various others, such as acibenzolar-S-methyl, anilazine, benthiavalicarb, blasticidin-S, chinomethionate, chloroneb, chlorothalonil, cyflufenamid, cymoxanil, dichlone, diclocymet, diclomezine, dicloran, diethofencarb, dimethomorph, flumorph, dithianon, ethaboxam, etridiazole, famoxadone, fenamidone, fenoxanil, fentin, ferimzone, fluazinam, fluopicolide, flusulfamide, fenhexamid, fosetyl-aluminium, hymexazol, iprovalicarb, cyazofamid, kasugamycin, mandipropamid, methasulfocarb, metrafenone, nicobifen, pencycuron, phthalide, polyoxins, probenazole, propamocarb, proquinazid, pyroquilon, quinoxyfen, quintozene, sulfur, tiadinil, triazoxide, tricyclazole, triforine, validamycin, zoxamide and glyphosate.

Another aspect of invention is related to the use of a compound of formula I, of a composition comprising at least one compound of formula I or of a fungicidal mixture comprising at least one compound of formula I in admixture with other fungicides, as described above, for controlling or preventing infestation of plants, harvested food crops or non-living materials by phytopathogenic microorganisms, preferably fungal organisms.

A further aspect of invention is related to a method of controlling or preventing an infestation of crop plants or of non-living materials by phytopathogenic or spoilage microorganisms or organisms potentially harmful to man, especially fungal organisms, which comprises the application of a compound of formula I as active ingredient to the plants, to parts of the plants or to the locus thereof, or to any part of the non-living materials. Controlling or preventing means reducing the infestation of crop plants or of non-living materials by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, which comprises the application of a compound of formula I, or an agrochemical composition which contains at least one of said compounds, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

A formulation [that is, a composition containing the compound of formula I] and, if desired, a solid or liquid adjuvant or monomers for encapsulating the compound of formula I, is prepared in a known manner, typically by intimately mixing and/or grinding the compound with extenders, for example solvents, solid carriers and, optionally, surface active compounds (surfactants).

The agrochemical formulations will usually contain from 0.1 to 99% by weight, preferably from 0.1 to 95% by weight, of the compound of formula I, 99.9 to 1% by weight, preferably 99.8 to 5% by weight, of a solid or liquid adjuvant, and from 0 to 25% by weight, preferably from 0.1 to 25% by weight, of a surfactant.

Advantageous rates of application are normally from 5g to 2kg of active ingredient (a.i.) per hectare (ha), preferably from 10g to 1 kg a.i./ha, most preferably from 20g to 600g a.i./ha. When used as seed drenching agent, convenient dosages are from 10mg to 1g of active substance per kg of seeds.

Whereas it is preferred to formulate commercial products as concentrates, the end user will normally use dilute formulations.

The following non-limiting examples illustrate the above-described invention in more detail.

### Example 1: This example illustrates the preparation of 2-Chloro-5-[4-chloro-2-methyl-5-(2,4,6-trifluoro-phfnyl)-imidazol-1-yl]-pyridine (Compound No.I.j.210)

### a) Preparation of (6-Chloro-pyridin-3-yl)-[1-(2,4,6-trifluoro-phenyl)-meth-(E)-ylidene]-amine

6-Chloro-pyridin-3-ylamine (5g) and 2,4,6-trifluoro-benzaldehyde (6.226g) are dissolved in toluene (190 ml). Subsequently, the mixture is stirred for 16.0 h at reflux in a Dean-Stark apparatus. The reaction mixture is evaporated under reduced pressure, to obtain 9.92 g of (6-chloro-pyridin-3-yl)-[1-(2,4,6-trifluoro-phenyl)-meth-(E)-ylidene]-amine. ¹H NMR (300Mhz, CDCl₃) 8.58ppm, 1H, s: 8.25ppm, 1 H, d, J=2.16Hz; 7.51 ppm, 1H, dd, J=2.47 and 8.36Hz; 7.36ppm, 1H, d, J=8.39Hz; 6.79ppm, 2H, t, J=8.69Hz.

### b) Preparation 2-Chloro-5-[5-(2,4,6-trifluoro-phenyl)-imidazol-1-yl]-pyridine

9.92 g of (6-Chloro-pyridin-3-yl)-[1-(2,4,6-trifluoro-phenyl)-meth-(E)-ylidene]-amine is dissolved in 120 ml of N,N-dimethyl-formamide and 100 ml of 1,2-dimethoxy-ethane. 10.73 g of toluenesulfonylmethyl isocyanide and 10.13g of anhydrous potassium carbonate are added, and the resulting reaction mixture is heated at 100°C for 90 minutes. After cooling down, the mixture is filtrated, the solvents evaporated, the resulting solid is adsorbed on Isolute^{®} HM-N and purified by chromatography column on silica gel, using a mixture of heptane / ethyl acetate 3:1 - 7:3 - 2:1 and 1:1 as successive eluents to obtain 9.320 g of an intermediate of molecular weight equal to 465.88 g/mol. This intermediate is dissolved in 80 ml of N,N-dimethyl-formamide and 70 ml of 1,2-dimethoxy-ethane, and to this solution is added 6.645 g of anhydrous potassium carbonate. The resulting reaction mixture is heated at 100°C for 24 h. After cooling down, the mixture is filtrated, the solvents evaporated, the resulting solid is adsorbed on lsolute^{®} HM-N and purified by chromatography column on silica gel, using a mixture of heptane / ethyl acetate 2:1 and 1:1 as successive eluents to obtain 4.782 g of 2-Chloro-5-[5-(2,4,6-trifluoro-phenyl)-imidazol-1-yl]-pyridine. ¹H NMR (300Mhz, CDCl₃) 8.26ppm, 1H, d, J=2.57Hz; 7.81 ppm, 1H, s; 7.48ppm, 1H, dd, J=2.7 and 8.44Hz; 7.38ppm, 1H, d, J=8.41Hz; 7.35ppm, 1 H, s; 6.69ppm, 2H, t, J=8Hz.

### c) Preparation of 5-[2-Bromo-5-(2,4,6-trifiuoro-phenyl)-imidazol-1-yl)-2-chloro-pyridine

A mixture of 3.290i g of 2-Chloro-5-[5-(2,4,6-trifluoro-phenyl)-imidazol-1-yl]-pyridine, 2.203 g of N-bromosuccinimide and 30 ml of chloroform is heated for 4 h to 80 °C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 4:1 as eluent to obtain 1.520 g of 5-[2-Bromo-5-(2,4,6-trifluoro-phenyl)-imidazol-1-yl]-2-chloro-pyridine as a pale yellow-orange solid. ¹H NMR (300Mhz, CDCl₃) 8.23ppm, 1 H, d, J=2.58Hz; 7.54ppm, 1H, dd, J=2.71 and 8.43Hz; 7.40ppm, 1 H, d, J=8.41 Hz; 7.27ppm, 1 H, s; 6.66ppm, 2H, t, J=7.85Hz.

### d) Preparation of 2-Chloro-5-[2-methyl-5-(2,4,6-trifluoro-phenyl)-imidazol-1-yl]-pyridine

5-[2-Bromo-5-(2,4,6-trifluoro-phenyl)-imidazol-1-yl]-2-chloro-pyridine (0.959 g) is dissolved in 50 ml of tetrahydrofurane. To this solution, 0.040 g of tetrakis(triphenylphosphine) Paladium is added, before refluxing the resulting mixture for 10 minutes. After this time, the oil bath is removed and, immediately, 3.7 ml of (trimethyl) Aluminium are added slowly. The reaction mixture is refluxed for exactly 95 minutes before being cooled down to 0°C. 2 ml of methanol are added dropwise (gas evolution) and after five minutes, lsolute^{®} HM-N is added and the solvents removed under reduced pressure. The residue is purified by chromatography on silica gel, using a mixture of heptane / ethyl acetate 1 : 4 as eluent, to deliver 0.218 g of 2-Chloro-5-[2-methyl-5-(2,4,6-trifluoro-phenyl)-imidazol-1-yl]-pyridine as a white solid. ¹H NMR (300Mhz, CDCl₃) 6.23ppm, 1 H, d, J=2.53Hz; 7.47ppm, 1H, dd, J=2.62 and 8.53Hz; 7.39ppm, 1H, d, J=8.4Hz; 7.20ppm, 1 H, s; 6.64ppm, 2H, t, J=7.46Hz; 2.38ppm, 3H, s. 0.312g of 2-Methyl-5-[2-methyl-5-(2,4,6-trifluoro-phenyl)-imidazol-1-yl]-pyridine are obtained as a by-product. ¹H NMR (300Mhz, CDCl₃) 8.23ppm, 1H, d, J=2.22Hz; 7.30ppm, 1H, dd, J=2.4 and 8.22Hz; 7.12ppm, 1 H, d, J=8.24Hz; 7.09ppm. 1 H, s; 6.53ppm, 2H, t, J=7.38Hz; 2.51 ppm, 3H, s; 2127ppm, 3H, s.

### e) Preparation of 2-Chloro-5-[4-chloro-2-methyl-5-(2,4,6-trifluoro-phenyl)-imidazol-1-yl]-pyridine (Compound No.I.j.210)

A mixture of 0.310 g of 2-Chloro-5-[2-methyl-5-(2,4,6-trifluoro-phenyl)-imidazol-1-yl]-pyidine, 0.134 g of N-chlorbsuceinimide and 3 ml of chloroform is heated for 3 h to 80 °C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 2:1 as eluent to obtain of 2-chloro-5-[4-chloro-2-methyl-5-(2,4,6-trifluoro-phenyl)-imidazol-1-yl]-pyridine. This white solid is dissolved in diethyl ether (10 ml), extracted twice with 5 ml of a 1 N sodium hydroxyde solution, the organic phase is dried over sodium sulfate, filtrated and evaporated under reduced pressure to obtain 0.195 g of 2-Chloro-5-[4-chloro-2-methyl-5-(2,4,6-trifluoro-phenyl)-imidazol-1-yl]-pyridine (Compound No.I.j.210) as a white solid. ¹H NMR (300Mhz, CDCl₃) 8.23ppm, 1H, d, J=2.49Hz; 7.47ppm, 1H, dd, J=2.63 and 8.43Hz; 7.40ppm, 1H, d, J=8.41 Hz; 6.68ppm, 2H, t, J=7.45Hz; 2.34ppm, 3H, s.

### Example 2: This example illustrates the preparation of 2-Chloro-5-[2,4-dichloro-5-(2,4,6-trifluoro-phonyl)-imidazol-1-yl]-pyridine (Compound No.I.j.209)

A mixture of 0.15 g of 2-Chloro-5-[5-(2,4,6-trifluoro-phenyl)-imidazol-1-yl]-pyridine, 0.164 g of N-chlorosuccinimide and 1.56 ml of chloroform is heated for 16 h to 80°C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 6 : 1 as eluent to obtain 0.136 g of 2-Chloro-5-[2,4-dichloro-6-(2,4,6-trifluom-phenyl)-imidazol-1-yl]-pyridine (Compound No.I.j.209). ¹H NMR (300Mhz, CDCl₃) 8.23ppm, 1H, d, J=2.7Hz; 7.54ppm, 1H, dd, J=2.7 and 8.5Hz; 7.43ppm, 1H, d, J=8.36Hz; 6.71 ppm, 2H, t, J=7.97Hz.

### Example 3: This example illustrates the preparation of 5-[4-Chloro-5-(2,6-difluoro-4-methoxyphenyl)-2-methyl-imidazol-1-yl]-2-methyl-pyridine (Compound No.I.m.225)

### a) Preparation of 5-[4-Chloro-2-methyl-5-(2,4,6-trifluoro-phenyl)-imidazol-1-yl]-2-methyl-pyridine (Compound No.I.j.225) .

A mixture of 0.448 g of 2-Methyl-5-[2-methyl-5-(2,4,6-trifluoro-phenyl)-imidazol-1-yl]-pyridine, 0.217 g of N-chlorpsuccinimide and 5 ml of chloroform is heated for 1.5 h to 80 °C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 1:1 as eluent to obtain of 5-[4-Chloro-2-methyl-5-(2,4,6-trifluoro-phenylfimidazol-1-yl]-2-methylpyridine (Compound No.I.j.225) contaminated by succinimide. This white solid is dissolved in diethyl ether (10 ml), extracted twice with 5 ml of a 1 N sodium hydroxyde solution, the organic phase is dried over sodium sulfate, filtrated and evaporated under reduced pressure to obtain 0.265 g of pure 5-[4-Chloro-2-methyl-5-(2,4,6-trifluoro-phenyl)-imidazol-1-yl]-2-methyl-pyridine (Compound No.I.j.225) as a white solid. ¹H NMR (300Mhz, CDCl₃) 8.32ppm, 1H, d, J=2.26Hz; 7.39ppm, 1H, dd, J=2.41 and 8.21Hz; 7.21ppm, 1 H, d, J=8.21 Hz; 6.65ppm, 2H, t, J=7.51Hz; 2.60ppm, 3H, s; 2.32ppm, 3H, s.

### b) Preparation of 5-[4-Chloro-5-(2,6-difiuoro-4-methoxy-phenyl)-2-methyl-imidazol-1-yl]-2-methyl-pyridine Compound No.I.m.225)

0.280 g of 5-[4-Chloro-2-methyl-5-(2,4,6-trifloro-phenyl)-imidazol-1-yl]-2-methyl-pyridine (Compound No.I.j225) are dissolved in 5 ml of tetrahydrofurane and the mixture is cooled down at 0°C. 305 µl of a solution of sodium methylate (30%) in methanol are added, and the reaction is stirred 16h at room temperature. 5 ml of ethyl acetate are added, followed by 5 ml of water, and the layers are separated. The aqueous phase is extracted two times with ethyl acetate, and the combined organic phases are dried over sodium sulfate, filtrated and evaporated under reduced pressure to obtain 0.260 g of 5-[4-Chloro-5-(2,6-difluoro-4-methoxy-phenyl)-2-methyl-imidazol-1-yl]-2-methyl-pyridine (Compound No.I.m.225). ¹H NMR (300Mhz, CDCl₃) 8.22ppm, 1 H, d, J=2.26Hz; 7.31 ppm, 1 H, dd, J=2.39 and 8.21 Hz; 7.10ppm, 1 H, d, J=8.21 Hz; 6.32ppm, 2H, d, J=9.1Hz; 3.69ppm, 3H, s; 2.50ppm, 3H, s; 2.22ppm. 3H, s.

### Example 4: This example illustrates the preparation of 5-[4-Chloro-5-(2,6-difluoro-4-methoxyphenyl)-2-methyl-imidazol-1-yl]-2-methoxy-pyridine (Compound No.I.m.220)

0.102 g of 2-Chloro-5-[4-chloro-2-methyl-5-(2,4,6-trifluoro-phenyl)-imidazol-1-yl]- pyridine (Compound No.I.j.210) are dissolved in 1.75 ml of tetrahydrofurane and the mixture is cooled down at 0°C. 132 µl of a solution of sodium methylate (30%) in methanol are added, and the reaction is stirred 16h at room temperature. 2 ml of ethyl acetate are added, followed by 3 ml of water, and the layers are separated. The aqueous phase is extracted two times with ethyl acetate, and the combined organic phases are dried over sodium sulfate, filtrated and evaporated under reduced pressure to obtain 0.099 g of 5-[4-Chloro-5-(2,5-difluoro-4-methoxy-phenyl)-2-methyl-imidazol-1-yl]-2-methoxy-pyridine (Compound No.I.m.220). ¹H NMR (300Mhz, CaCl₃) 7.90ppm, 1H, d, J=2.6Hz; 7.28ppm, 1H, dd, J=2.72 and 8.72Hz; 6.67ppm, 1 H, d, J=8.78Hz; 6.33ppm, 2H, d, J=9.1 Hz; 3.86ppm, 3H, s; 3.69ppm, 3H, s; 2.21 ppm, 3H, s.

### Example 5: This example illustrates the preparation of 2-Chloro-5-[4-chloro-5-(2,6-difiuoro-4-methoxy-phenyl)-2-methyl-imidazol-1-yl]-pyridine (Compound No.I.m.210)

0.089 g of 5-[4-Chloro-5-(2,6-difluoro-4-methoxy-phenyl)-2-methyl-imidazol-1-yl]-2-methoxy-pyridine (Compound No.I.m.220) is dissolved in 1 ml of phosphoroxychloride and refluxed for 24 hours. The solution is poured onto cold water and stirred for 30 minutes, then a 1 M solution of sodium hydroxyde is added to reach neutrality. The aqueous solution is extracted three times with dichloromethane, the combined organic fractions are dried onto sodium sulfate and the solvent is removed under reduced pressure. The crude mixture is purified by chromatography column on silica gel, using a mixture of heptane / ethyl acetate 3 : 1 as eluent to obtain 0.041 g of 2-Chloro-5-14-chloro-5-(2,6-dffluoro-4-methoxy-phenyl)-2-methyl-imidazol-1-yl]-pyridine (Compound No.I.m.210). ¹H NMR (300Mhz, CDCl₃) 8.15ppm, 1H, d, J=2.7Hz; 7.39ppm, 1 H, dd, J=2.7 and 8.4Hz; 7.31 ppm, 1H, d, J=8.4Hz; 6.34ppm, 2H, d, J=9.25Hz; 3.71 ppm, 3H, s; 2.25ppm, 3H, s.

Table 1 below illustrates examples of individual compounds of formula I according to the invention.

**Table 1: individual compounds of formula I according to the invention**

| **Compound No.** | **R¹** | **R²** | **R³** |
|---|---|---|---|
| 001 | F | phenyl | F |
| 002 | CH₃ | phenyl | F |
| 003 | F | phenyl | Cl |
| 004 | Cl | phenyl | Cl |
| 005 | CH₃ | phenyl | Cl |
| 006 | F | 3-fluorophenyl | F |
| 007 | CH₃ | 3-fluorophenyl | F |
| 008 | F | 3-fluorophenyl | Cl |
| 009 | Cl | 3-fluorophenyl | Cl |
| 010 | CH₃ | 3-fluorophenyl | Cl |
| 011 | F | 4-fluorophenyl | F |
| 012 | CH₃ | 4-fluorophenyl | F |
| 013 | F | 4-fluorophenyl | Cl |
| 014 | Cl | 4-fluorophenyl | Cl |
| 015 | CH₃ | 4-fluorophenyl | Cl |
| 016 | F | 3-chlorophenyl | F |
| 017 | CH₃ | 3-chlorophenyl | F |
| 018 | F | 3-chlorophenyl | Cl |
| 019 | Cl | 3-chlorophenyl | Cl |
| 020 | CH₃ | 3-chlorophenyl | Cl |
| 021 | F | 4-chlorophenyl | F |
| 022 | CH₃ | 4-chlorophenyl | F |
| 023 | F | 4-chlorophenyl | Cl |
| 024 | Cl | 4-chlorophenyl | Cl |
| 025 | CH₃ | 4-chlorophenyl | Cl |
| 026 | F | 3-bromophenyl | F |
| 027 | CH₃ | 3-bromophenyl | F |
| 028 | F | 3-bromophenyl | Cl |
| 029 | Cl | 3-bromophenyl | Cl |
| 030 | CH₃ | 3-bromophenyl | Cl |
| 031 | F | 4-bromophenyl | F |
| 032 | CH₃ | 4-bromophenyl | F |
| 033 | F | 4-bromophenyl | Cl |
| 034 | Cl | 4-bromophenyl | Cl |
| 035 | CH₃ | 4-bromophenyl | Cl |
| 036 . | F | m-tolyl | F |
| 037 | CH₃ | m-tolyl | F |
| 038 | F | m-tolyl | Cl |
| 039 | Cl | m-tolyl | Cl |
| 040 | CH₃ | m-tolyl | Cl |
| 041 | F | p-tolyl | F |
| 042 | CH₃ | p-tolyl | F |
| 043 | F | p-tolyl | Cl |
| 044 | Cl | p-tolyl | Cl |
| 045 | CH₃ | p-tolyl | Cl |
| 046 | F | 3-trifluoromethylphenyl | F |
| 047 | CH₃ | 3-trifluoromeihyiphenyl | F |
| 048 | F | 3-trifluoromethylphenyl | Cl |
| 049 | Cl | 3-trifluoromethylphenyl | Cl |
| 050 | CH₃ | 3-trifluoromethylphenyl | Cl |
| 051 | F | 4-trifluoromethylphenyl | F |
| 052 | CH₃ | 4-trifluoromethylphenyl | F |
| 053 | F | 4-trtfluoromethylphenyl | Cl |
| 054 | Cl | 4-trifluoromethylphenyl | Cl |
| 055 | CH₃ | 4-trifluoromethylphenyl | Cl |
| 056 | F | 3-methoxyphenyl | F |
| 057 | CH₃ | 3-methoxyphenyl | F |
| 058 | F | 3-methoxyphenyl | Cl |
| 059 | Cl | 3-methoxyphenyl | Cl |
| 060 | CH₃ | 3-methoxyphenyl | Cl |
| 061 | F | 4-methoxyphenyl | F |
| 062 | CH₃ | 4-methoxyphenyl | F |
| 063 | F | 4-methoxyphenyl | Cl |
| 064 | Cl | 4-methoxyphenyl | Cl |
| 065 | CH₃ | 4-methoxyphenyl | Cl |
| 066 | F | 3-trifluoromethoxyphenyl | F |
| 067 | CH₃ | 3-trifluoromethoxyphenyl | F |
| 068 | F | 3-trifluoromethoxyphenyl | Cl |
| 069 | Cl | 3-trifluoromethoxyphenyl | Cl |
| 070 | CH₃ | 3-trifluoromethoxyphenyl | Cl |
| 071 | F | 4-trifluoromethoxyphenyl | F |
| 072 | CH₃ | 4-trifluoromethoxyphenyl | F |
| 073 | F | 4-tritluoromethoxyphenyl | Cl |
| 074 | Cl | 4-trifluommethoxyphenyl | Cl |
| 075 | CH₃ | 4-tritluoromethoxyphenyl | Cl |
| 076 | F | 3-cyanophenyl | F |
| 077 | CH₃ | 3-cyanophenyl | F |
| 078 | F | 3-cyanophenyl | Cl |
| 079 | Cl | 3-cyanophenyl | Cl |
| 080 | CH₃ | 3-cyanophenyl | Cl |
| 081 | F | 4-cyanophenyl | F |
| 082 | CH₃ | 4-cyanophenyl | F |
| 083 | F | 4-cyanophenyl | Cl |
| 084 | Cl | 4-cyanophenyl | Cl |
| 085 | CH₃ | 4-cyanophenyl | Cl |
| 086 | F | 3,4-difluorophenyl | F |
| 087 | CH₃ | 3,4-difluorophenyl | F |
| 088 | F | 3,4-difluorophenyl | Cl |
| 089 | Cl | 3,4-difluorophenyl | Cl |
| 090 | CH₃ | 3,4-difluorophenyl | Cl |
| 091 | F | 3,4-dichlorophenyl | F |
| 092 | CH₃ | 3,4-dichlorophenyl | F |
| 093 | F | 3,4-dichlorophenyl | Cl |
| 094 | Cl | 3,4-dichforophenyl | Cl |
| 095 | CH₃ | 3,4-dichlorophenyl | Cl |
| 096 | F | 3,4-dimethylphenyl | F |
| 097 | CH₃ | 3,4-dimethylphenyl | F |
| 098 | F | 3,4-dimethylphenyl | Cl |
| 099 | Cl | 3,4-dimethylphenyl | Cl |
| 100 | CH₃ | 3,4-dimethylphenyl | Cl |
| 101 | F | 3,4-dimethoxyphenyl | F |
| 102 | CH₃ | 3,4-dimethoxyphenyl | F |
| 103 | F | 3,4-dimethoxyphenyl | Cl |
| 104 | Cl | 3,4-dimethoxyphenyl | Cl |
| 105 | CH₃ | 3,4-dimethoxyphenyl | Cl |
| 106 | F | 3-chloro-4-fluorophenyl | F |
| 107 | CH₃ | 3-chloro-4-fluorophonyl | F |
| 108 | F | 3-chloro-4-fluorophenyl | Cl |
| 109 | Cl | 3-chloro-4-fluorophenyl | Cl |
| 110 | CH₃ | 3-chloro-4-fluorophenyl | Cl |
| 111 | F | 3-chloro-4-methylphenyl | F |
| 112 | CH₃ | 3-chloro-4-methylphenyl | F |
| 113 | F | 3-chloro-4-methylphenyl | Cl |
| 114 | Cl | 3-chloro-4-methylphenyl | Cl |
| 115 | CH₃ | 3-chloro-4-methylphenyl | Cl |
| 116 | F | 3-chloro-4-methoxyphenyl | F |
| 117 | CH₃ | 3-chtoro-4-methoxyphenyl | F |
| 118 | F | 3-chloro-4-methoxyphenyl | Cl |
| 119 | Cl | 3-chloro-4-methoxyphenyl | Cl |
| 120 | CH₃ | 3-chloro-4-methoxyphenyl | Cl |
| 121 | F | 4-chloro-3-fluorophenyl | F |
| 122 | CH₃ | 4-chloro-3-fluorophenyl | F |
| 123 | F | 4-chloro-3-fluorophenyl | Cl |
| 124 | Cl | 4-chloro-3-fluorophenyl | Cl |
| 125 | CH₃ | 4-chloro-3-fluorophenyl | Cl |
| 126 | F | 4-chloro-3-methylphenyl | F |
| 127 | CH₃ | 4-chloro-3-methylphenyl | F |
| 128 | F | 4-chloro-3-methylphenyl | Cl |
| 129 | Cl | 4-chlorc-3-methylphenyl | Cl |
| 130 | CH₃ | 4-chloro-3-methylphenyl | Cl |
| 131 | F | 4-chloro-3-methoxyphenyl | F |
| 132 ' | CH₃ | 4-chloro-3-methoxyphenyl | F |
| 133 | F | 4-chloro-3-methoxyphenyl | Cl |
| 134 | Cl | 4-chloro-3-methoxyphenyl | Cl |
| 135 | CH₃ | 4-chloro-3-methoxyphenyl | Cl |
| 136 | F | 3-fluoro-4-methoxyphenyl | F |
| 137 | CH₃ | 3-fluoro-4-methoxyphenyl | F |
| 138 | F | 3-fluoro-4-methoxyphenyl | Cl |
| 139 | Cl | 3-fluoro4-methoxyphenyl | Cl |
| 140 | CH₃ | 3-fluoro-4-methoxyphenyl | Cl |
| 141 | F | 3-fluoro-4-methylphenyl | F |
| 142 | CH₃ | 3-fluoro-4-methylphenyl | F |
| 143 | F | 3-tluoro-4-methylphenyl | Cl |
| 144 | Cl | 3-fluoro-4-methylphenyl | Cl |
| 145 | CH₃ | 3-fluoro-4-methylphenyl | Cl |
| 146 | F | 4-fluoro-3-methoxyphenyl | F |
| 147 | CH₃ | 4-fluoro-3-methoxyphenyl | F |
| 148 | F | 4-fluoro-3-methoxyphenyl | Cl |
| 149 | Cl | 4-fluoro-3-methoxyphenyl | Cl |
| 150 | CH₃ | 4-fluoro-3-methoxyphenyl | Cl |
| 151 | F | 4-fluoro-3-methylphenyl | F |
| 152 | CH₃ | 4-fluom-3-methylphenyl | F |
| 153 | F | 4-fluoro-3-methylphenyl | Cl |
| 154 | Cl | 4-fluoro-3-methylphenyl | Cl |
| 155 | CH₃ | 4-fluoro-3-methylphenyl | Cl |
| 156 | F | 3-methoxy-4-methylphenyl | F |
| 157 | CH₃ | 3-methoxy-4-methylphenyl | F |
| 158 | F | 3-methoxy-4-methylphenyl | Cl |
| 159 | Cl | 3-methoxy-4-methylphenyl | Cl |
| 160 | CH₃ | 3-methoxy-4-methylphenyl | Cl |
| 161 | F | 4-methoxy-3-methylphenyl | F |
| 162 | CH₃ | 4-methoxy-3-methytphenyl | F |
| 163 | F | 4-methoxy-3-methylphenyl | Cl |
| 164 | Cl | 4-methoxy-3-methylphenyl | Cl |
| 165 | CH₃ | 4-methoxy-3-methylphenyl | Cl |
| 166 | F | naphth-2-yl | F |
| 167 | CH₃ | naphth-2-yl | F |
| 168 | F | naphth-2-yl | Cl |
| 169 | Cl | naphth-2-yl | Cl |
| 170 | CH₃ | naphth-2-yl | Cl |
| 171 | F | pyridin-2-yl | F |
| 172 | CH₃ | pyridin-2-yl | F |
| 173 | F | pyridin-2-yl | Cl |
| 174 | Cl | pyridin-2-yl | Cl |
| 175 | CH₃ | pyridin-2-yl | Cl |
| 176 | F | pyridin-3-yl | F |
| 177 | CH₃ | pyridin-3-yl | F |
| 178 | F | pyridin-3-yl | Cl |
| 179 | Cl | pyridin-3-yl | Cl |
| 180 | CH₃ | pyridin-3-yl | Cl |
| 181 | F | pyridin-4-yl | F |
| 182 | CH₃ | pyridin-4-yl | F |
| 183 | F | pyridin-4-yl | Cl |
| 184 | Cl | pyridin-4-yl | Cl |
| 185 | CH₃ | pyridin-4-yl | Cl |
| 186 | F | 6-chloropyridin-2-yl | F |
| 187 | CH₃ | 6-chloropyridin-2-yl | F |
| 188 | F | 6-chloropyridin-2-yl | Cl |
| 189 | Cl | 6-chloropyridin-2-yl | Cl |
| 190 | CH₃ | 6-chloropyridin-2-yl | Cl |
| 191 | F | 6-fluoropyridin-2-yl | F |
| 192 | CH₃ | 6-fluoropyridin-2-yl | F |
| 193 | F | 6-fluoropyridin-2-yl | Cl |
| 194 | Cl | 6-fluoropyridin-2-yl | Cl |
| 195 | CH₃ | 6-fluoropyridin-2-yl | Cl |
| 196 | F | . 6-methoxypyridin-2-yl | F |
| 197 | CH₃ | 6-methoxypyridin-2-yl | F |
| 198 | F | 6-methoxypyridin-2-yl | Cl |
| 199 | Cl | 6-methoxypyridin-2-yl | Cl |
| 200 | CH₃ | 6-methoxypyridin-2-yl | Cl |
| 201 | F | 6-methylpyridin-2-yl | F |
| 202 | CH₃ | 6-methylpyridin-2-yl | F |
| 203 | F | 6-methylpyridin-2-yl | Cl |
| 204 | Cl | 6-methylpyridin-2-yl | Cl |
| 205 | CH₃ | 6-methylpyridin-2-yl | Cl |
| 206 | F | 6-chloropyridin-3-yl | F |
| 207 | CH₃ | 6-chloropyridin-3-yl | F |
| 208 | F | 6-chloropyridin-3-yl | Cl |
| 209 | Cl | 6-chloropyridin-3-yl | Cl |
| 210 | CH₃ | 6-chloropyridin-3-yl | Cl |
| 211 | F | 6-fluoropyridin-3-yl | F |
| 212 | CH₃ | 6-fluoropyridin-3-yl | F |
| 213 | F | 6-fluoropyridin-3-yl | Cl |
| 214 | Cl | 6-fluoropyridin-3-yl | Cl |
| 215 | CH₃ | 6-fluoropyridin-3-yl | Cl |
| 216 | F | 6-methoxypyridin-3-yl | F |
| 217 | CH₃ | 6-methoxypyridin-3-yl | F |
| 218 | F | 6-methoxypyridin-3-yl | Cl |
| 219 | Cl | 6-methoxypyridin-3-yl | Cl |
| 220 | CH₃ | 6-methoxypyridin-3-yl | Cl |
| 221 | F | 6-methylpyridin-3-yl | F |
| 222 | CH₃ | 6-methylpyridin-3-yl | F |
| 223 | F | 6-methylpyridin-3-yl | Cl |
| 224 | Cl | 6-methylpyridin-3-yl | Cl |
| 225 | CH₃ | 6-methylpyridin-3-yl | Cl |
| 226 | F | 2-chloropyridin-4-yl | F |
| 227 | CH₃ | 2-chloropyridin-4-yl | F |
| 228 | F | 2-chloropyridin-4-yl | Cl |
| 229 | Cl | 2-chloropyridin-4-yl | Cl |
| 230 | CH₃ | 2-chloropyridin-4-yl | Cl |
| 231 | F | 2-fluoropyridin-4-yl | F |
| 232 | CH₃ | 2-fluoropyridin-4-yl | F |
| 233 | F | 2-fluoropyridin-4-yl | Cl |
| 234 | Cl | 2-fluoropyridin-4-yl | Cl |
| 235 | CH₃ | 2-fluoropyridin-4-yl | Cl |
| 236 | F | 2-methoxypyridin-4-yl | F |
| 237 | CH₃ | 2-methoxypyridin-4-yl | F |
| 238 | F | 2-methoxypyridin-4-yl | Cl |
| 239 | Cl | 2-methoxypyridin-4-yl | Cl |
| 240 | CH₃ | 2-methoxypyridin-4-yl | Cl |
| 241 | F | 2-methylpyridin-4-yl | F |
| 242 | CH₃ | 2-methylpyridin-4-yl | F |
| 243 | F | 2-methylpyridin-4-yl | Cl |
| 244 | Cl | 2-methylpyridin-4-yl | Cl |
| 245 | CH₃ | 2-methylpyridin-4-yl | Cl |
| 246 | F | 5-chlorothiophen-2-yl | F |
| 247 | CH₃ | . 5-chlorothiophen-2-yl | F |
| 248 | F | 5-chlorothiophen-2-yl | Cl |
| 249 | Cl | 5-chlorothiophen-2-yl | Cl |
| 250 | CH₃ | 5-chlorothlophen-2-yl | Cl |
| 251 | F | 5-bromothiophen-2-yl | F |
| 252 | CH₃ | 5-bromothiophen-2-yl | F |
| 253 | F | 5-bromothiophen-2-yl | Cl |
| 254 | Cl | 5-bromothiophen-2-yl | Cl |
| 255 | CH₃ | 5-bromothiophen-2-yl | Cl |
| 256 | F | 5-methoxythiophen-2-yl | F |
| 257 | CH₃ | 5-methoxythlophen-2-yl | F |
| 258 | F | 5-methoxythiophen-2-yl | Cl |
| 259 | Cl | 5-methoxythiophen-2-yl | Cl |
| 260 | CH₃ | 5-methoxythiophen-2-yl | Cl |
| 261 | F | quinolin-2-yl | F |
| 262 | CH₃ | quinolin-2-yl | F |
| 263 | F | quinolin-2-yl | Cl |
| 264 | Cl | quinolin-2-yl | Cl |
| 265 | CH₃ | quinolin-2-yl | Cl |
| 266 | F | quinolin-3-yl | F |
| 267 | CH₃ | quinolin-3-yl | F |
| 268 | F | quinolin-3-yl | Cl |
| 269 | Cl | quinolin-3-yl | Cl |
| 270 | CH₃ | quinolin-3-yl | Cl |
| 271 | F | 4-methoxyquinolin-2-yl | F |
| 272 | CH₃ | 4-methoxyquinolin-2-yl | F |
| 273 | F | 4-methoxyquinolin-2-yl | Cl |
| 274 ' | Cl | 4-methoxyquinolin-2-yl | Cl |
| 275 | CH₃ | 4-methoxyquinolin-2-yl | Cl |
| 276 | F | 4-methylquinolin-2-yl | F |
| 277 | CH₃ | 4-methylquinolin-2-yl | F |
| 278 | F | 4-methylquinolin-2-yl | Cl |
| 279 | Cl | 4-methylquinolin-2-yl | Cl |
| 280 | CH₃ | 4-methylquinolin-2-yl | Cl |

where
a) 280 compounds of formula (I.a): 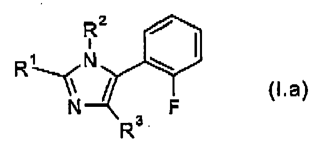 wherein R¹, R² and R³ are as defined in Table 1.
b) 280 compounds of formula (I.b): 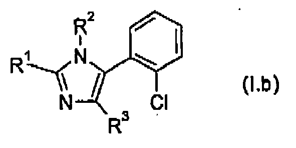 wherein R¹, R² and R³ are as defined in Table 1.
c) 280 compounds of formula (I.c): 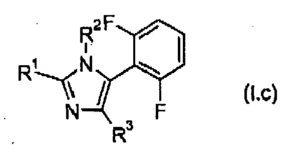 wherein R¹, R² and R³ are as defined in Table 1.
d) 280 compounds of formula (I.d): 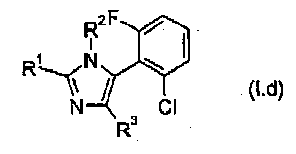 wherein R¹, R² and R³ are as defined in Table 1.
e) 280 compounds of formula (I.e): 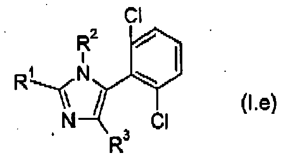 wherein R¹, R² and R³ are as defined in Table 1.
f) 280 compounds of formula (I.f): 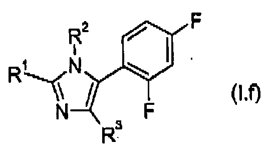 wherein R¹, R² and R³ are as defined in Table 1.
g) 280 compounds of formula (I.g): 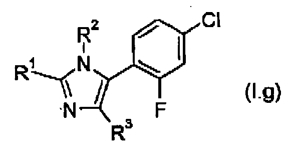 wherein R¹, R² and R³ are as defined in Table 1.
h) 280 compounds of formula (I.h): 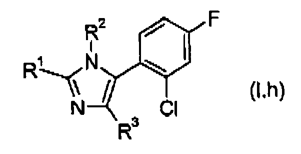 wherein R¹, R² and R³ are as defined in Table 1.
i) 280 compounds of formula (I.i): 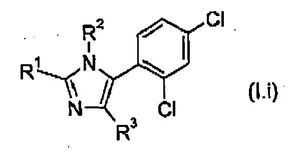 wherein R¹, R² and R³ are as defined in Table 1.
j) 280 compounds of formula (I.j): 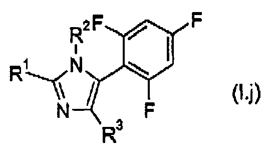 wherein R¹, R² and R³ are as defined in Table 1.
k) 280 compounds of formula (I.k): 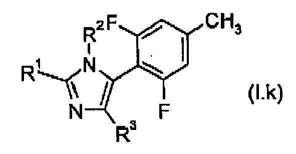 wherein R¹, R² and R³ are as defined in Table 1.
l) 280 compounds of formula (I.l): 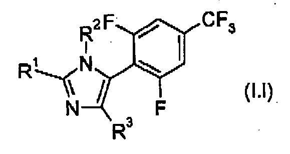 wherein R¹, R² and R³ are as defined in Table 1.
m) 280 compounds of formula (I.m): 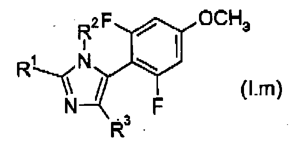 wherein R¹, R² and R³ are as defined in Table 1.
n) 280 compounds of formula (I.n): 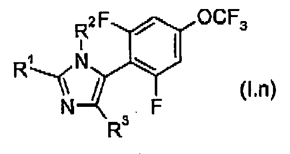 wherein R¹, R² and R³ are as defined in Table 1.
o) 280 compounds of formula (I.o): 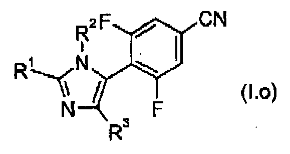 wherein R¹, R² and R³ are as defined in Table 1.
p) 280 compounds of formula (I.p): 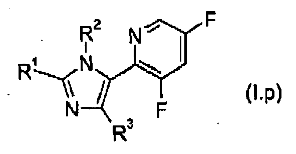 wherein R¹, R² and R³ are as defined in Table 1.
q) 280 compounds of formula (I.q): 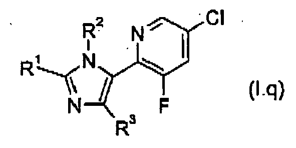 wherein R¹, R² and R³ are as defined in Table 1.
r) 280 compounds of formula (I.r): 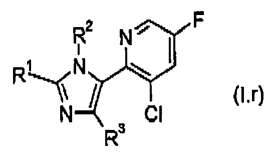 wherein R¹, R² and R³ are as defined in Table 1.
s) 280 compounds of formula (I.s): 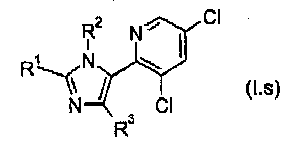 wherein R¹, R² and R³ are as defined in Table 1.
t) 280 compounds of formula (I.t): 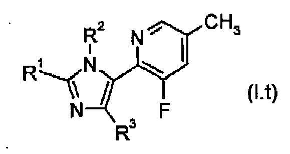 wherein R¹, R² and R³ are as defined in Table 1.
u) 280 compounds of formula (I.u): 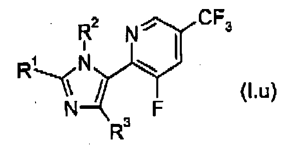 wherein R¹, R² and R³ are as defined in Table 1.
v) 280 compounds of formula (I.v): 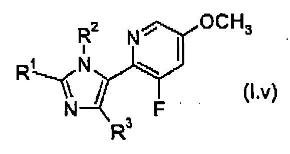 wherein R¹, R² and R³ are as defined in Table 1.
w) 280 compounds of formula (I.w): 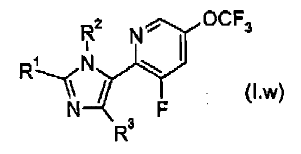 wherein R¹, R² and R³ are as defined in Table 1.
x) 280 compounds of formula (I.x): 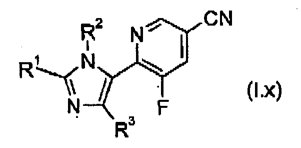 wherein R¹, R² and R³ are as defined in Table 1.
y) 280 compounds of formula (I.y): 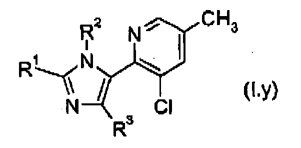 wherein R¹, R² and R³ are as defined in Table 1.
z) 280 compounds of formula (I.z): 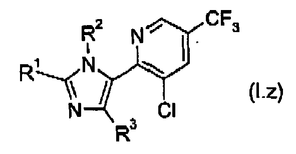 wherein R¹, R² and R³ are as defined in Table 1.
aa) 280 compounds of formula (I.aa): 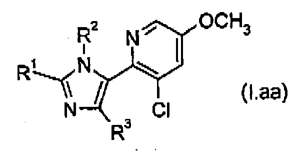 wherein R¹, R² and R³ are as defined in Table 1.
ab) 280 compounds of formula (I.ab): 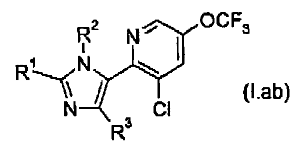 wherein R¹, R² and R³ are as defined in Table 1.
ac) 280 compounds of formula (I.ac): 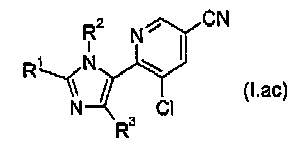 wherein R¹, R² and R³ are as defined in Table 1.

Throughout this description, temperatures are given in degrees Celsius, "m.p." means melting point, "NMR" means nuclear magnetic resonance spectrum; and "%" is percent by weight, unless corresponding concentrations are indicated in other units.

The following abbreviations are used throughout this description:

| | | | |
|---|---|---|---|
| m.p. = | melting point | br = | broad |
| s = | singlet | dd = | doublet of doublets |
| d = | doublet | dt = | doublet of triplets |
| t = | triplet | q = | quartet |
| m = | multiplet | ppm = | parts per million |

Table 2 shows selected NMR data, all with CDCl₃ as the solvent (unless otherwise stated, no attempt is made to list all characterising data in all cases) for compounds of Table

**Table 2: Selected NMR data for compounds of Table 1**

| Compound Number | ¹H-NMR data (ppm/number of H's/multiplicity) |
|---|---|
| I.j.209 | 8.23ppm, 1 H, d, J=2.70Hz; 7.54ppm, 1 H, dd, |
| | J=2.70 and 8.50Hz; 7.43ppm, 1 H, d, J=8.36Hz; |
| | 6.71ppm, 2H, t, J=7.97Hz |
| I.j.210 | 8.23ppm, 1H, d, J=2.49Hz; 7.47ppm, 1H, dd, |
| | J=2.63 and 8.43Hz; 7.40ppm, 1 H, d, J=8.41 Hz; |
| | 6.68ppm, 2H, t, J=7.45Hz; 2.34ppm, 3H, s |
| I.j.225 | 8.32ppm, 1H, d, J=2.26Hz; 7.39ppm, 1 H, dd, |
| | J=2.41 and 8.21 Hz; 7.21 ppm, 1 H, d, J=8.21 Hz; |
| | 6.65ppm, 2H, t, J=7.51Hz; 2.60ppm, 3H, s; |
| | 2.32ppm, 3H, s |
| I.j.269 | 8.70ppm, 1H, d, J=2.27Hz; 8.17ppm, 1H, d, |
| | J=8.37Hz; 8.06ppm, 1 H, s; 7.86ppm, 2H, m; |
| | 7.67ppm, 1H, t, J=7.34Hz; 6.64ppm, 2H, t, |
| | J=7.82Hz |
| I.j.270 | 8.61ppm, 1H, d, J=2.24Hz; 8.08ppm, 1H, d, |
| | J=8.40Hz; 7.92ppm, 1H, d, J=2.13Hz; 7.77ppm, |
| | 1H, d, J=7.54Hz; 7.76ppm, 1H, t, J=8.25Hz; |
| | 7.58ppm, 1H, t, J=7.57Hz; 6.52ppm, 2H, t, J=7.79Hz; 2.29ppm, 3H, s |
| I.m.210 | ¹H NMR (300Mhz, CDCl₃) 8.15ppm, 1H, d, |
| | J=2.7Hz; 7.39ppm, 1 H, dd, J=2.7 and 8.4Hz; |
| | 7.31 ppm, 1H, d, J=8.4Hz; 6.34ppm, 2H, d, |
| | J=9.25Hz; 3.71 ppm, 3H, s; 2.25ppm, 3H, s. |
| I.m.220 | ¹H NMR (300Mhz, CDCl₃) 7.90ppm, 1H, d, |
| | J=2.6Hz; 7.28ppm, 1 H, dd, J=2.72 and 8.72Hz; |
| | 6.67ppm, 1 H, d, J=8.78Hz; 6.33ppm, 2H, d, |
| | J=9.1Hz; 3.86ppm, 3H, s; 3.69ppm, 3H, s; |
| | 2.21 ppm, 3H, s. |
| I.m.225 | 8.22ppm, 1H, d, J=2.26Hz; 7.31 ppm, 1H, dd, |
| | J=2.39 and 8.21 Hz; 7.10ppm, 1H, d, J=8.21Hz; |
| | 6.32ppm, 2H, d, J=9.1 Hz; 3.69ppm, 3H, s; |
| | 2.50ppm, 3H, s; 2.22ppm, 3H, s. |

The compounds according to the present invention can be prepared according to the above-mentioned reaction schemes, in which, unless otherwise stated, the definition of each variable is as defined above for a compound of formula (I).

### Biological examples

### Alternaria solani / tomato / preventive (Action against Alternaria on tomato)

4 weeks old tomato plants cv. Roter Gnom are treated with the formulated test compound in a spray chamber. Two days after application tomato plants are inoculated by spraying a spore suspension on the test plants. After an incubation period of 4 days at 22 °C / 18 °C and 95% r. h. in a greenhouse the disease incidence is assessed.

Compounds I.j.209, I.j.210, I.j.225, I.j.269, I.j.270, I.m.210, I.m.220 and I.m.225 according to the invention at 200 ppm inhibit fungal infestation in this test to at least 80 %, while under the same conditions untreated control plants are infected by the phytopathogenic fungi to over 80%.

### Botrytis cinerea / tomato / preventive (Action against Botrytis on tomato

4 weeks old tomato plants cv. Roter Gnom are treated with the formulated test compound in a spray chamber. Two days after application tomato plants are inoculated by spraying a spore suspension on the test plants. After an incubation period of 3 days at 20 °C and 95% r. h. in a greenhouseithe disease incidence is assessed.

Compounds I.j.209, I.j.210, I.j.270, I.m.210, I.m.220 and I.m.225 according to the invention at 200 ppm inhibit furigal infestation in this test to at least 80 %, while under the same conditions untreated control plants are infected by the phytopathogenic fungi to over 80 %.

### Puccinia recondita / wheat / preventive (Action against brown rust on wheat)

1 week old wheat plants cv. Arina are treated with the formulated test compound in a spray chamber. One day after application wheat plants are inoculated by spraying a spore suspension (1 x 105 uredospores/ml) on the test plants. After an incubation period of 1 day at 20 °C and 95% r. h: plants are kept for 10 days 20 °C / 18 °C (day/night) and 60% r.h. in a greenhouse. The disease incidence is assessed 11 days after inoculation.

Compounds I.j.210, I.j.270, I.m.210, I.m.220 and I.m.225 according to the invention at 200 ppm inhibit fungal infestation in this test to at least 80 %, while under the same conditions untreated control plants are infected by the phytopathogenic fungi to over 80 %.

### Magnaporthe grisea (Pyricularia oryzae) / rice / preventive (Action against rice blast)

3 weeks old rice plants cv. Koshihikari are treated with the formulated test compound in a spray chamber. Two days after application rice plants are inoculated by spraying a spore suspension (1 x 10⁵ conidia/ml) on the test plants. After an incubation period of 6 days at 25°C and 95% r. h. the disease incidence is assessed.

Compounds I.j.209, I.j.210, I.j.225, I.j.270, I.m.210, I.m.220 and I.m.225 according to the invention at 200 ppm inhibit fungal infestation in this test to at least 80 %, while under the same conditions untreated control plants are infected by the phytopathogenic fungi to over 80 %.

### Pyrenophora teres (Helminthosporium teres) / barley / preventive (Action against net blotch on barley)

1-week-old barley plants cv. Regina are treated with the formulated test compound in a spray chamber. Two days after application barley plants are inoculated by spraying a spore suspension (2.6 x 10⁴ conidia/ml) on the test plants. After an incubation period of 4 days at 20 °C and 95% r. the disease incidence is assessed.

Compounds I.j.209, I.j.210, I.j.269, I.j.270, I.m.210, I.m.220 and I.m.225 according to the invention at 200 ppm inhibit fungal infestation in this test to at least 80 %, while under the same conditions untreated control plants are infected by the phytopathogenic fungi to over 80 %.

### Septoria tritici / wheat / preventive (Action against Septoria leaf spot on wheat)

2 weeks old wheat plants cv. Riband are treated with the formulated test compound in a spray chamber. One day after application wheat plants are inoculated by spraying a spore suspension (10⁶ conidia/ml) on the test plants. After an incubation period of 1 day at 22 °C / 21 °C and 95% r. h. plants are kept at 22 °C / 21 °C and 70% r.h. in a greenhouse. The disease incidence is assessed 16 - 18 days after inoculation.

Compounds I.j.209, I.j.210, I.j.225, I.j.269. I.j.270, I.m.210, I.m.220 and I.m.225 according to the invention at 200 ppm inhibit fungal infestation in this test to at least 80 %, while under the same conditions Untreated control plants are infected by the phytopathogenic fungi to over 80 %.

### Uncinula necator / grape / preventive (Action against powdery mildew on grape)

5 weeks old grape seedlings cv. Gutedel are treated with the formulated test compound in a spray chamber. One day after application grape plants are inoculated by shaking plants infected with grape powdery mildew above the test plants. After an incubation period of 7 days at 24 °C / 22 °C and 70% r. h. under a light regime of 14/10 h (light/dark) the disease incidence is assessed.

Compounds I.j.210, I.j.225, I.j.269, I.j.270, I.m.210, I.m.220 and I.m.225 according to the invention at 200 ppm inhibit fungal infestation in this test to at least 80 %, while under the same conditions untreated control plants are infected by the phytopathogenic fungi to over 80%.

## Claims

1. A compound of formula I: 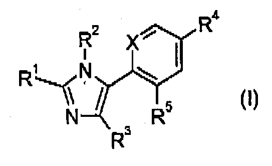 wherein
R¹ is halogen, C₁-G₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, hydroxyl, C₁-C₄alkoxy, OR⁶, C₁-C₄haloalkoxy or cyano;
R⁵ is halogen;
R⁶ is hydrogen, C₃-C₇ cycloalkyl, C₃-C₁₀ alkylcycloalkyl, C₁-C₆ haloalkyl, C₂-C₆ alkenyl, C₂-C₆ haloalkenyl, C₃-C₇ cycloalkenyl, C₂-C₆ alkynyl, C₂-C₆ haloalkynyl or C₂-C₆ alkyloxyalkyl;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;
provided that
when X is C(R), R² cannot be an optionally substituted aryl.

2. The compound according to claim 1 wherein R¹ is halogen, C₁-C₃alkyl or C₁-C₃haloalkyl.

3. The compound according to either claims 1 or 2 wherein R² is an optionally substituted phenyl, naphthyl, thienyl, pyridyl or quinolyl.

4. The compound according to any one of claims 1 to 3 wherein R³ is fluoro, chloro or bromo.

5. The compound according to any one of claims 1 to 4 wherein R⁴ is hydrogen, halogen, C₁-C₄alkyl, C₁-C₄haloalkyl, C₁-C₄alkoxy, OR⁸, C₁-C₄haloalkoxy or cyano.

6. The compound according to any one of claims 1 to 5 wherein R⁵ is fluoro, chloro, bromo or iodo.

7. The compound according to any one of claims 1 to 6 wherein R⁶ is hydrogen, C₃-C₇ cycloalkyl, C₃-C₁₀ alkylcycloalkyl, C₁-C₆ haloalkyl, C₂-C₆ alkenyl, C₃-C₇ cycloalkenyl, C₂-C₆ alkynyl or C₂-C₆ alkyloxyalkyl.

8. The compound according to any one of claims 1 to 7 wherein X is N, C(H), C(halogen), C(C₁-C₄alkyl), C(C₁-C₄haloalkyl), C(C₁-C₄alkoxy) or C(C₁-C₄haloalkoxy).

9. The compound according to any one of claims 1 to 8 wherein
R¹ is fluoro, chloro, bromo, iodo, C₁-C₂alkyl or C₁-C₂haloalkyl;
R² is an optionally substituted phenyl, naphtyl, thienyl, pyridyl or quinolyl;R³ is fluoro, chloro or bromo;
R⁴ is hydrogen, fluoro, chloro, bromo, C₁-C₃alkyl, C₁-C₃haloalkyl, C₁-C₃alkpxy, C₁-C₃haloalkoxy or cyano;
R⁵ is fluoro, chloro or bromo; and
X is N, C(H), C(Cl), C(F), C(Br), C(I), C(C₁-C₃alkyl), C(C₁-C₃haloalkyl), C(C₁-C₃alkoxy) or C(C₁-C₃haloalkoxy).

10. The compound according to any one of claims 1 to 9 wherein
R¹ is fluoro, chloro, bromo, methyl or ethyl;
R² is phenyl, 3-fluorophenyl, 4-fluorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl, 3-methoxyphenyl, 4-methoxyphenyl, 3-trifluoromethoxyphenyl, 4-trifluoromethoxyphenyl, 3-cyanophenyl, 4-cyanophenyl, 3,4-difluorophenyl, 3,4-dichlorophenyl, 3,4-dimethylphenyl, 3,4-dimethoxyphenyl, 3-chloro-4-fluorophenyl, 3-chlaro-4-methylphenyl, 3-chloro-4-methoxyphenyl, 4-chloro-3-fluorophenyl, 4-chloro-3-methylphenyl, 4-chloro-3-methoxyphenyl, naphth-2-yl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, pyridin-2-yl, pyridin-3-yl, pyridin-4-yl, 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 5-chlorothiophen-2-yl, 5-bromothlophen-2-yl, 5-mothoxythlophen-2-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl, C₁-C₂alkoxy, C₁-C₂haloalkoxy or cyano;
R⁵ is fluoro or chloro; and
X is N, C(H), C(Cl); C(F), C(Br), C(C₁-C₂alkyl), C(C₁-C₂heloalkyl), C(C₁-C₂alkoxy) or C(C₁-C₂haloalkoxy).

11. The compound according to any one of claims 1 to 10 wherein
R¹ is fluoro, chloro, methyl or ethyl;
R² is 4-fluorophenyl, 4-chlorophenyl, 4-bromophenyl, p-tolyl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl or C₁-C₂alkoxy,
R⁵ is fluoro or chloro; and
X is N, C(H), C(Cl) or C(F).

12. The compound according to any one of claims 1 to 11 wherein
R¹ is chloro or methyl;
R² is 6-chloropyridin-3-yl, 6-methylpyridin-3-yl, 6-methoxypyridin-3-yl or quinolin-3-yl;
R³ is chloro;
R⁴ is fluoro or methoxy; and
R⁵ is fluoro; and
X is C(F).

13. A compound selected from
2-chloro-5-[2,4-dichloro-5-(2,4,6-trifluoro-ophenyl)-imidazol-1-yl]-pyridine;
2-chloro-5-[4-chloro-2-methyl-5-(2,4,6-trifluoro-phenyl)-imidazol-1-yl]-pyridine;
5-[4-chloro-2-methyl-5-(2,4,6-trifluoro-phenyl)-imidazol-1-yl]-2-methoxy-pyridine;
2-[4-chloro-2-methyl-5-(2,4,6-trifluoro-phenyl)-imidazol-1-yl]-quinoline;
2-[4-chloro-2-methyl-5-(2,4,6-trifluoro-phenyl)-imidazol-1-yl]-4-methoxy-quinoline;
3-[4-chloro-2-methyl-5-(2,4,6-trifluoro-phenyl)-imidazol-1-yl]-quinoline;
3-[2,4-dichloro-5-(2,4,6-trifluoro-phenyl)-imidazol-1-yl]-quinoline;
3-[2,4-dichloro-5-(2,6-difluoro-4-methoxy-phenyl)-imidazol-1-yl]-quinoline;
2-[4-chloro-5-(2,6-difluoro-4-methoxy-phenyl)-2-methyl-imidazol-1-yl]-4-methoxy-quinoline;
3-[4-chloro-5-(2,6-difluoro-4-mathoxy-phenyl)-2-methyl-imidazol-1-yl]-quinoline;
2-chloro-5-[4-chloro-5-(2,6-difluoro-4-methoxy-phenyl)-2-methyl-imidazol-1-yl]-pyridine;
2-chloro-5-[2,4-dichloro-5-(2,6-difluoro-4-methoxy-phenyl)-imidezol-1-yl]-pyridine;
5-[4-chloro-5-(2,6-difluoro-4-methoxy-phenyl)-2-methyl-imidazol-1-yl]-2-methoxy-pyridine;
5-[4-Chloro-5-(2,6-difluoro-4-methoxy-phenyl)-2-methyl-imidazol-1-yl]-2-methyl-pyridine;
3,5-dichloro-2-[5-chloro-3-(6-chloro-pyridin-3-yl)-2-methyl-3H-imidazol4-yl]-pyridine; and
2-[4-chloro-5-(3,5-dichloro-pyridin-2-yl)-2-methyl-imidazol-1-yl]-quinoline.

14. A process for the preparation of a compound of formula I. 1, 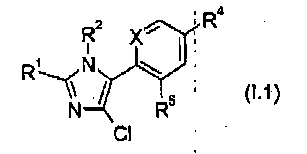 wherein R², R⁴, R⁵ and X are as defined for compound of formula I and R¹ is C₁-C₄alkyl or C₁-C₄haloalkyl, which comprises reacting a compound of formula II, 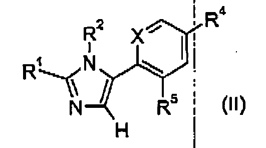 wherein R², R⁴, R⁵ and X are as defined for compound of formula I and R¹ is C₁-C₄alkyl or C₁-C₄haloalkyl, with N-chlorosuccinimide or molecular chlorine.

15. A process for the preparation of a compound of formula 1.2, 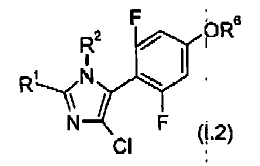 wherein R² and R⁶ are as defined for compound of formula I, and R¹ is C₁-C₄alkyl, which comprises reacting a compound of formula I.3, 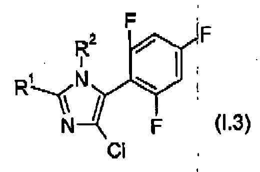 wherein R² is as defined for compound of formula I, and R¹ is C₁-C₄alkyl, with a reagent of formula NaOR⁶, wherein R⁶ is as defined for compound of formula I.

16. A process for the preparation of a compound of formula II, 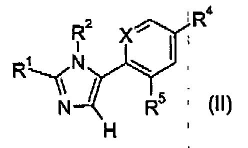 wherein R², R⁴, R⁵ and X are as defined for compound of formula I and R¹ is C₁-C₄alkyl, which comprises reacting a compound of formula III, 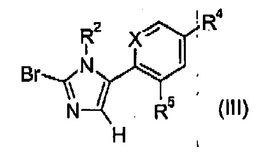 wherein R², R⁴, R⁵ and X are as defined for compound of formula I, with a reagent of formula (R¹)₃Al, wherein R¹ is C₁-C₄alkyl in the presence of a transition metal catalyst.

17. A process for the preparation of a compound of formula II, 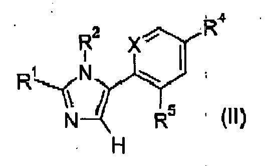 wherein R², R⁴, R⁵ and X are as defined for compound of formula I and R¹ is C₁-C₄alkyl or C₁-C₄haloalkyl, which comprises reacting a compound of formula IV, 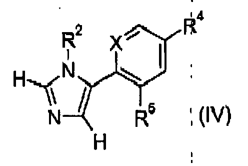 wherein R², R⁴, R⁵ and X are as defined for compound of formula I, with a strong base followed by a reagent of formula R¹ Hal, wherein R¹ is C₁-C₄alkyl or C₁-C₄haloalkyl and Hal is halogen.

18. A process for the preparation of a compound of formula I, 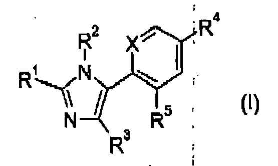 wherein R², R⁴, R⁵ and X are as defined for compound of formula I and R¹ is halogen, preferably chlorine or bromine, which comprises reacting a compound of formula IV, 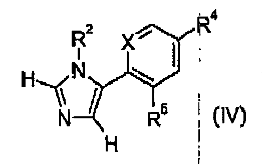 wherein R², R⁴, R⁵ and X are as defined for compound of formula I, with N-chlorosuccinimide N-bromosuccinimide, molecular chlorine or molecular bromine.

19. A process for the preparation of a compound of formula III, 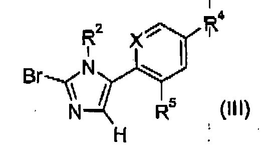 wherein R², R⁴, R⁵ and X are as defined for compound of formula I, which comprises reacting a compound of formula IV, 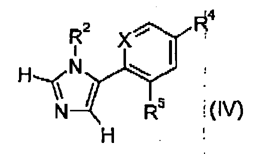 wherein R², R⁴, R⁵ and X are as defined for compound of formula I, with N-bromosuccinimide.

20. A fungicidal composition for controlling or protecting against phytopathogenic microorganisms, comprising as active ingredient at least one compound as defined in any one of claims 1 to 13, in free form or in agrochemically usable salt form, and at least one adjuvant.

21. The composition according to claim 20, which comprises at least one additional fungicidally active compound, preferably selected from the group consisting of azoles, pyrimidinyl carbinoles, 2-amino-pyrimidines, morpholines, anilinopyrimidines, pyrroles, phenylamides, benzimidazoles, dicarboximides, carboxamides, strobilurines, dithiocarbamates, N-halomethylthiotetrahydrophthalimides, copper-compounds, nitrophenols, organo-phosphorus-derivatives, pyridazines, triazolopyrimidines, carboxamides or benzamides.

22. The use of a compound as defined in any one of claims 1 to 13 for controlling or preventing infestation of plants, harvested food crops, seeds or non-living materials by phytopathogenic microorganisms.

23. A method of controlling or preventing an infestation of crop plants, harvested food crops or non-living materials by phytopathogenic or spoilage microorganisms or organisms potentially harmful to man, which comprises the application of a compound as defined in any one of claims 1 to 13, as active ingredient to the plant, to parts of the plants or to the locus thereof, to seeds or to any part of the non-living materials.

24. The method according to claim 23, wherein the phytopathogenic microorganisms are fungal organisms.
